# FASCICULE DE BREVET EUROPEEN

(11) **EP 1 758 587 B1**
(45) Date de publication et mention de la délivrance du brevet: **08.09.2010**
(21) Numéro de dépôt: 05775762.7
(22) Date de dépôt: 01.06.2005
(51) Int. Cl.: A61K 31/573, A61K 31/59, A61P 17/06

(54) **UTILISATION D'UNE COMPOSITION PHARMACEUTIQUE COMPRENANT DU CALCITRIOL ET DU PROPIONATE DE CLOBETASOL POUR LE TRAITEMENT DU PSORIASIS**
VERWENDUNG EINER PHARMAZEUTISCHEN ZUSAMMENSETZUNG MIT CALCITRIOL UND CLOBETASOLPROPIONAT ZUR BEHANDLUNG VON SCHUPPENFLECHTE
USE OF A PHARMACEUTICAL COMPOSITION COMPRISING CALCITRIOL AND CLOBETASOL PROPIONATE FOR THE TREATMENT OF PSORIASIS

(30) Priorité: 17.06.2004 FR 0406608
(43) Date de publication de la demande: 07.03.2007
(73) Titulaire: Galderma S.A., 6330 Cham (CH)
(72) Inventeur: ANDRES, Philippe, F-06530 Peymeinade (FR)
(74) Mandataire: Allab, Myriam
(86) Numéro de dépôt international: PCT/FR2005/001348
(87) Numéro de publication internationale: WO 2006/008354

(56) Documents cités:
- WO-A-00/64450
- WO-A-02/34235
- WO-A-2004/054588
- US-A- 6 126 949
- LAHFA M ET AL: "CALCITRIOL OINTMENT AND CLOBETASOL PROPIONATE CREAM: A NEW REGIMEN FOR THE TREATMENT OF PLAQUE PSORIASIS" EUROPEAN JOURNAL OF DERMATOLOGY, XX, XX, vol. 13, no. 3, mai 2003 (2003-05), pages 261-265, XP009016887 ISSN: 1167-1122
- LAMBA S ET AL: "Combination therapy with vitamin D analogues" BRITISH JOURNAL OF DERMATOLOGY, XX, XX, vol. 144, no. Supplement 58, avril 2001 (2001-04), pages 27-32, XP002253887 ISSN: 0007-0963
- KRAGBALLE K ET AL: "Efficacy of once-daily treatment regimens with calcipotriol/betamethasone dipropionate ointment and calcipotriol ointment in psoriasis vulgaris." THE BRITISH JOURNAL OF DERMATOLOGY. JUN 2004, vol. 150, no. 6, juin 2004 (2004-06), pages 1167-1173, XP001205154 ISSN: 0007-0963
- ANONYMOUS: "Dovobet Oinment" INTERNET ARTICLE, [Online] 1 novembre 2004 (2004-11-01), XP002314150 Extrait de l'Internet: URL:http://emc.medicines.org.uk/emc/assets /c/html/displaydoc.asp?documentid=8851#COM POSITION> [extrait le 2005-01-19]
- DATABASE PHAR [Online] PJB Publications Ltd, UK; ANONYMOUS: "Daivobet" XP002314151 extrait de STN Database accession no. AN: 23423

## Description

La présente invention se rapporte à une composition pharmaceutique comprenant, dans un milieu pharmaceutiquement acceptable, du calcitriol et du propionate de clobétasol, et à l'utilisation de cette composition pour la préparation d'un médicament destiné au traitement du psoriasis.

Le psoriasis est une maladie inflammatoire chronique de la peau qui touche environ 3% de la population française. Cette maladie se manifeste par des plaques rouges recouvertes de squames blanchâtres qui se détachent de la peau. Les plaques de psoriasis se localisent souvent aux coudes, genoux, cuir chevelu et bas du dos, mais peuvent atteindre toutes les autres parties du corps comme le visage, les mains, les pieds, les plis, les ongles. Le psoriasis peut survenir à tout âge, mais les premières poussées apparaissent la plupart du temps dans la deuxième ou troisième décade. C'est une maladie chronique dont l'évolution est imprévisible : aux phases de récidives succèdent des phases de rémission. Si cette maladie met rarement en danger la vie d'une personne, elle a en revanche un fort impact sur sa qualité de vie, étant donné son aspect inesthétique et sa chronicité. Dans le psoriasis, certaines personnes souffrent d'une seule plaque de psoriasis localisée dans une région bien précise du corps tandis que d'autres sont sujettes à un psoriasis étendu à l'ensemble du corps.

Plus spécifiquement, l'invention se rapporte à une composition pharmaceutique comprenant du calcitriol et du propionate de clobétasol dans dans les quantités de la revendication 1, de façon à ce qu'un effet synergique entre les deux principes actifs soit observé dans le traitement du psoriasis.

L'association de plusieurs principes actifs peut se révéler avantageuse car elle permet de réduire les doses des actifs administrés, et ainsi de réduire les effets secondaires de ces actifs. Cependant de telles associations ne sont pas utilisées d'une manière classique dans le traitement des affections dermatologiques, étant donné les problèmes de stabilité chimique et d'interactions entre les actifs associés dans une même formulation.

Le calcitriol est la forme naturelle et active de la vitamine D. Elle est notamment utilisée pour les carences en calcium et réguler son homéostasie dans l'organisme.

Le propionate de clobetasol, ou 17-propionante de clobetasol, est un corticostéroïde. Le mécanisme d'action des corticostéroïdes est attribué à leur inhibition des processus inflammatoires (Lange K et al, Skin Pharmacol Appl Skin Physiol 13(2) :93-103 (2000)). Dans la suite de la description, propionate de clobetasol et 17-propionate de clobetasol seront utilisés indifféremment.

Le document US 4,610,978 décrit des compositions pour application topique dans le traitement des maladies dermatologiques telles que par exemple le psoriasis, comprenant du calcitriol. Ces compositions peuvent en outre contenir un corticostéroïde tel que par exemple l'hydrocortisone ou la dexaméthasone.

Le document WO 00/64450 décrit une composition pharmaceutique pour usage dermique contenant un analogue de la vitamine D et un corticostéroïde. Les exemples donnés concernent plus particulièrement des compositions comprenant du calcipotriol (analogue de la vitamine D) et de la bétaméthasone (corticostéroïde). La comparaison des mesures de l'efficacité sur des patients atteints de psoriasis d'une composition comprenant du calcipotriol seul, du bétaméthasone seul ou l'association des deux actifs montrent que l'effet obtenu par l'association correspond à un effet additif. Ainsi au regard de ce document, l'homme du métier ne pouvait nullement imaginer que l'association d'un analogue de la vitamine D avec un corticostéroide puisse présenter un effet synergique. En outre ce document ne décrit pas spécifiquement l'association de propionate de clobétasol avec du calcitriol.

Dans le document FR 2 848 454, la Demanderesse a découvert que l'association du calcitriol avec du propionate de clobétasol permettait d'obtenir un effet synergique dans le traitement de certaines affections dermatologiques telles que le psoriasis, la dermatite atopique, la dermatite de contact et la dermatite séborrhéique.

Le document Lahfta M. et al., European Jornal of Dermatology, 13(3), 2003, 261, décrit une étude de traitement du psoriasis consistant en l'administration d'une crème de propionate de clobétasole à 0.05% le matin et d'un onguent de calcitriol à 3 µg/g le soir pour 2-4 semaines, suivi par l'administration du calcitriol seul jusqu'à la semaine 12.

La Demanderesse a maintenant découvert, de La Demanderesse a maintenant découvert, de manière surprenante, que des quantités particulières de propionate de clobetasol et de calcitriol, permettaient d'obtenir un effet synergique particulièrement intéressant dans le traitement du psoriasis, de façon à ce que les compositions pharmaceutiques comprenant lesdits composés ne soient appliquées qu'une seule fois par jour sur la ou les parties de la peau affectée(s) par le psoriasis. Cet effet avantageux est obtenu grâce à l'application des deux actifs au même moment sur leur site d'action.

La présente invention a donc pour objet une composition pharmaceutique destinée au traitement du psoriasis **caractérisée en ce qu**'elle comprend du propionate de clobétasol et du calcitriol en une quantité appropriée pour une application une fois par jour sur la ou les parties de la peau affectée(s) par le psoriasis.

La composition de l'invention comprend 3 µg de calcitriol par gramme de composition et 250 µg de propionate de clobétasol par gramme de composition.

La composition de l'invention comprend en outre un ou plusieurs excipients pharmaceutiques adaptés pour une application topique.

Selon un mode de réalisation avantageux, la composition de l'invention se présente sous la forme d'un gel, d'une crème, d'une pommade, d'une lotion ou d'une solution.

De préférence les crèmes peuvent être formulées à partir d'un mélange d'huile minérale, ou d'un mélange de cire d'abeille et d'eau qui s'émulsifie instantanément, dans lequel on additionne le calcitriol et le propionate de clobétasol, dissout dans une petite quantité d'huile tel que l'huile d'amande.

De préférence les lotions peuvent être préparées en dissolvant le calcitriol et le propionate de clobétasol dans un alcool à masse moléculaire élevée, tel que le polyéthylène glycol.

Les pommades peuvent être formulées en mélangeant une solution de calcitriol et de propionate de clobétasol dans une huile tel que l'huile d'amande dans de la paraffine chauffée, puis en laissant refroidir le mélange.

Les gels peuvent être préparés de préférence en dispersant ou en dissolvant le calcitriol et le propionate de clobétasol dans un rapport approprié, dans un gel de type carbomère, poloxamère ou cellulosique.

D'autres ingrédients peuvent encore être additionnés à la composition topique, tels que des conservateurs, par exemple la DL-alpha-tocophérol, ou des parfums, si nécessaire.

La présente invention concerne également l'utilisation d'une association de propionate de clobétasol et de calcitriol pour la fabrication d'un médicament destiné au traitement du psoriasis, ledit médicament contenant les quantités revendiquées de propionate de clobétasol et de calcitriol afin d'être appliqué une fois par jour sur la ou les parties de la peau affectée (s) par le psoriasis.

Les quantités respectives de propionate de clobétasol et de calcitriol sont telles que définies ci-dessus.

En outre, le médicament comprend un ou plusieurs excipients pharmaceutiques adaptés pour une application topique, et se présente sous la forme d'un gel, d'une crème, d'une pommade, d'une lotion ou d'une solution.

### Description des figures :

Les Figures 1 et 2 représentent la moyenne du TSS en fonction du temps des sept compositions testées et décrites dans l'exemple 1 ci-après.
Les figures 3 et 4 représentent le nombre de sujets dont la lésion a été guérie en fonction du temps des sept compositions testées et décrites dans l'exemple 1 ci-après.

L'exemple ci-dessous illustre l'invention, il ne la limite en aucune façon.

### EXEMPLE 1 : Évaluation de l'efficacité de quatre compositions différentes de l'invention dans le « test modifié de Dumas-Scholtz : traitement en mini-zones dans le psoriasis »

L'étude comparative décrite ci-dessous, « *monocentrique, randomisée, intra-individuelle à investigateur masqué, et contrôlée par un comparateur actif* », a été conduite pour évaluer l'efficacité et la tolérance locale de quatre compositions objets de l'invention, comprenant différentes concentrations de propionate de clobétasol et de calcitriol, en comparaison avec trois compositions non objets de l'invention.

Les quatre compositions objets de l'invention sont les suivantes :
- composition comprenant respectivement 250 µg de propionate de clobétasol et 9 µg de calcitriol par gramme de composition, à savoir plus particulièrement une crème comprenant 250 µg/g de propionate de clobétasol en association avec un onguent comprenant 9 µg/g de calcitriol ;
- composition comprenant respectivement 250 µg de propionate de clobétasol et 3 µg de calcitriol par gramme de composition, à savoir plus particulièrement une crème comprenant 250 µg/g de propionate de clobétasol en association avec un onguent comprenant 3 µg/g de calcitriol (Silkis ®) ;
- composition comprenant respectivement 500 µg de propionate de clobétasol et 9 µg de calcitriol par gramme de composition, à savoir plus particulièrement une crème comprenant 500 µg/g de propionate de clobétasol (Dermoval ®) en association avec un onguent comprenant 9 µg/g de calcitriol ;
- composition comprenant respectivement 500 µg de propionate de clobétasol et 3 µg de calcitriol par gramme de composition, à savoir plus particulièrement une crème comprenant 500 µg/g de propionate de clobétasol

(Dermoval ®) en association avec un onguent comprenant 3 µg/g de calcitriol (Silkis ®).

Les trois compositions non objets de l'invention sont les suivantes :
- composition commercialisée sous la dénomination « Daivobet^{®} », constituée d'un onguent de la combinaion 500 µg/g de diproprionate de bétaméthasone (corticostéroïde) et 50 µg/g de calcipotriol en tant que substances actives ;
- composition comprenant 250 µg de propionate de clobétasol par gramme de composition en association avec le véhicule de Silkis (onguent associant vaseline et huile de paraffine) ;
- composition comprenant 500 µg de propionate de clobétasol par gramme de composition (Dermoval ®) en association avec un le véhicule de Silkis (onguent vaseline/huile de paraffine).

L'objectif de l'étude est d'identifier et de choisir les concentrations de calcitriol et de propionate de clobétasol qui présentent, après application du traitement pendant 21 jours sur 29 sujets atteints de psoriasis (65,5% d'hommes et 34,5% de femmes), un profil d'efficacité supérieur à ceux d'une composition de Daivobet^{®} et de compositions comprenant uniquement du propionate de clobétasol, ledit propionate de clobétasol étant présent à une même concentration que celle d'une composition comprenant une association de propionate de clobétasol et de calcitriol.

### Méthodologie :

Les sept compositions testées ont été aléatoirement appliquées à sept emplacements sur une ou plusieurs plaques psoriasiques de sévérité identique localisées sur les jambes (face antérieure du tibia exclue), sur le tronc ou sur les bras.

Les différentes compositions ont été appliquées en condition normale d'utilisation c'est à dire sans occlusion, une seule fois par jour, sauf le dimanche, pour un total de 18 fois en tout (soit 21 jours d'experimentation).

Les applications des compositions sont effectuées de la façon suivante : on applique dans un premier temps uniquement le propionate de clobétasol, et lorsque ce dernier a complètement pénétré, on applique dans les mêmes conditions le calcitriol ou le véhicule de Silkis sans période de latence entre les deux applications.

Des évaluations cliniques *(scores d'érythème, d'infiltration, de desquamation et de guérison)* ont été faites lors de la sélection des patients, à leur inclusion (Baseline) ainsi qu'aux jours 4, 8, 11, 15, 18 et 22 de l'étude. Des événements indésirables ont également été enregistrés.

Le critère principal mesuré est l'aire sous la courbe (« Area Under Curve », AUC) calculée du jour 1 au jour 22 (évaluation finale) sur le TSS (Total Sum Score = *somme des scores individuels d'érythème, d'infiltration et de desquamation)*.

L'analyse statistique a été exécutée par l'intermédiaire d'une analyse de la variance incluant *"Sujet", "Zone" et "Traitement"* suivie d'un test de Tukey pour des comparaisons multiples.

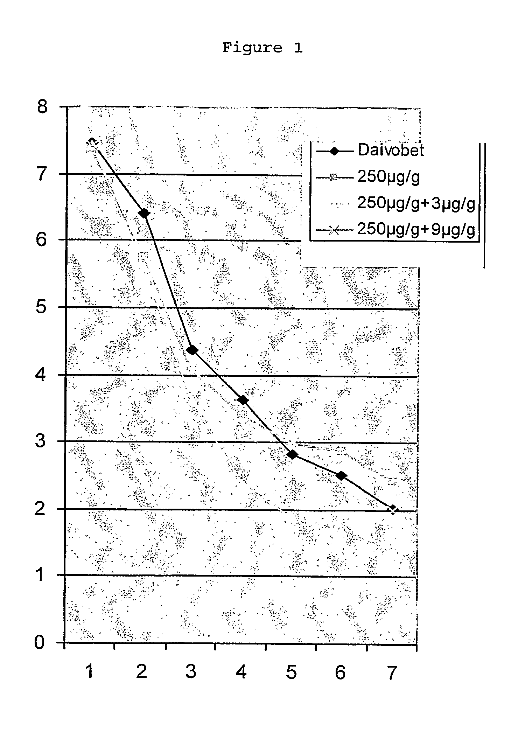

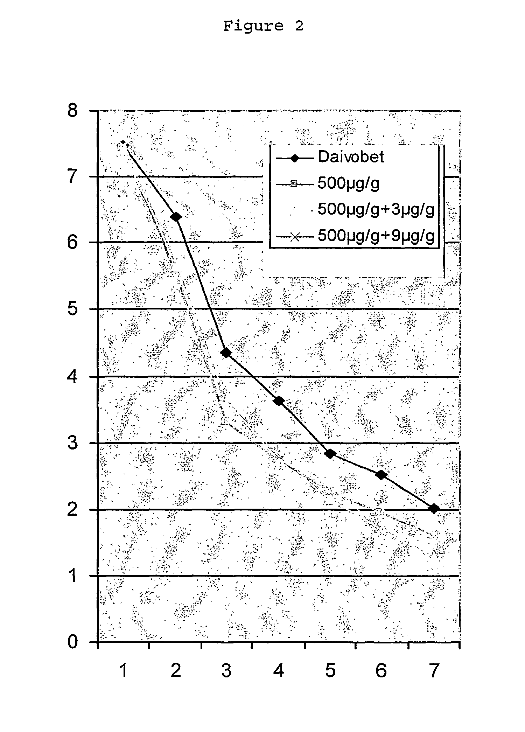

Les figures 1 et 2 représentent la moyenne du TSS (Total sum score = addition des scores individuels pour l'érythème, la desquamation et l'infiltration) en fonction du temps de sept compositions différentes objets ou non de l'invention et décrites dans cet exemple.

Dans la figure 1, la courbe reliée par des losanges noirs correspond au traitement par une composition « Daivobet® ».

La courbe reliée par des carrés gris foncé correspond au traitement par une composition comprenant 250 µg de propionate de clobétasol par gramme de composition.

La courbe reliée par des triangles blancs correspond au traitement par une composition de l'invention comprenant respectivement 250 µg de propionate de clobétasol et 3 µg de calcitriol par gramme de composition.

La courbe reliée par des croix noires correspond au traitement par une composition de l'invention comprenant respectivement 250 µg de propionate de clobétasol et 9 µg de calcitriol par gramme de composition.

Dans la figure 2, la courbe reliée par des losanges noirs correspond au traitement par une composition « Daivobet® ».

La courbe reliée par des carrés gris foncé correspond au traitement par une composition comprenant 500 µg de propionate de clobétasol par gramme de composition.

La courbe reliée par des triangles blancs correspond au traitement par une composition de l'invention comprenant respectivement 500 µg de propionate de clobétasol et 3 µg de calcitriol par gramme de composition.

La courbe reliée par des croix noires correspond au traitement par une composition de l'invention comprenant respectivement 500 µg de propionate de clobétasol et 9 µg de calcitriol par gramme de composition.

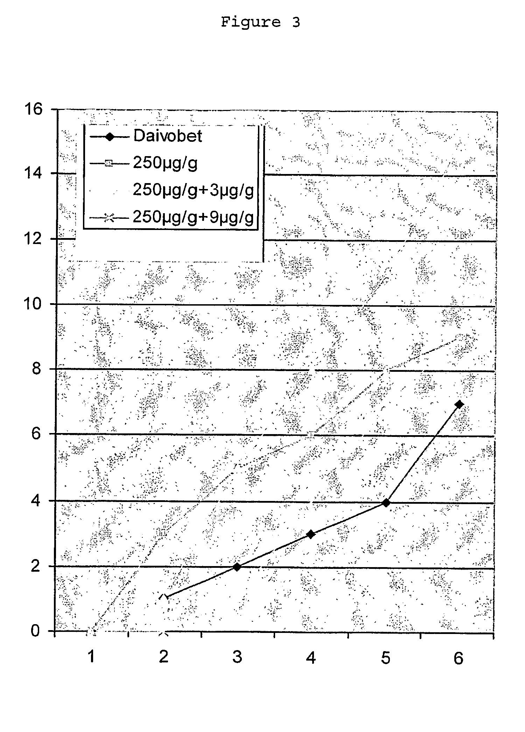

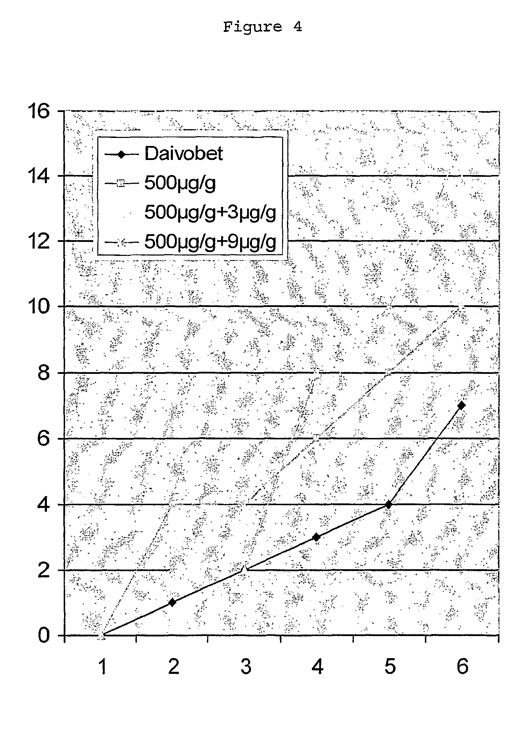

Les figures 3 et 4 représentent le nombre de sujets dont la lésion a été guérie en fonction du temps de sept compositions différentes objets ou non de l'invention et décrites dans cet exemple.

Dans la figure 3, la courbe reliée par des losanges noirs correspond au traitement par une composition « Daivobet® ».

La courbe reliée par des carrés gris foncé correspond au traitement par une composition comprenant 250 µg de propionate de clobétasol par gramme de composition.

La courbe reliée par des triangles blancs correspond au traitement par une composition de l'invention comprenant respectivement 250 µg de propionate de clobétasol et 3 µg de calcitriol par gramme de composition.

La courbe reliée par des croix noires correspond au traitement par une composition de l'invention comprenant respectivement 250 µg de propionate de clobétasol et 9 µg de calcitriol par gramme de composition.

Dans la figure 4, la courbe reliée par des losanges noirs correspond au traitement par une composition « Daivobet® » .

La courbe reliée par des carrés gris foncé correspond au traitement par une composition comprenant 500 µg de propionate de clobétasol par gramme de composition.

La courbe reliée par des triangles blancs correspond au traitement par une composition de l'invention comprenant respectivement 500 µg de propionate de clobétasol et 3 µg de calcitriol par gramme de composition.

La courbe reliée par des croix noires correspond au traitement par une composition de l'invention comprenant respectivement 500 µg de propionate de clobétasol et 9 µg de calcitriol par gramme de composition.

### Résultats :

29 sujets au total ont été randomisés et inclus dans l'étude. Aucun d'eux n'a été exclu des analyses.

Efficacité : Les *AUCs* du *TSS* sont illustrés dans le tableau 1 ci-dessous.

**Tableau 1 :**

| **Compositions testées** | **AUC du TSS** |
|---|---|
| Propionate de clobétasol 250 µg/g + calcitriol 3 µg/g | 66,96 |
| Propionate de clobétasol 500 µg/g + calcitriol 3 µg/g | 68,98 |
| Propionate de clobétasol 500 µg/g + véhicule de calcitriol | 69,60 |
| Propionate de clobétasol 500 µg/g + calcitriol 9 µg/g | 71,12 |
| Propionate de clobétasol 250 µg/g + calcitriol 9 µg/g | 77,64 |
| Propionate de clobétasol 250 µg/g + véhicule de calcitriol | 82,65 |
| Daivobet^{®} | 84,20 |

Conclusion :

Les résultats obtenus, en termes *d'AUC du TSS (critère primaire)*, pour une composition comprenant du propionate de clobétasol 250 µg/g et du calcitriol 3 µg/g (AUC = 66.96), sont statistiquement supérieurs à ceux obtenus pour :
- une composition comprenant du propionate de clobétasol 250 µg/g et un véhicule (*AUC* = 82.65) et,
- pour une composition Daivobet^{®} (*AUC* = 84.20).

On a observé les mêmes résultats pour *l'AUC moyen de l'infiltration*. Dans les autres critères, on a observé la même tendance sans atteindre de signification statistique. Innocuité : *Quatorze événements indésirables* ont été rapportés, aucun d'eux n'était relié au traitement ou n'a mené à une *sortie prématurée d'essai*.

Dans ces conditions d'étude, une composition comprenant 250 µg/g de propionate de clobétasol et 3 µg/g de calcitriol a montré des résultats significativement meilleurs en efficacité que le propionate de clobétasol 250 µg/g seul et que le Daivobet^{®}. De plus, cette association a montré des résultats d'efficacité semblables au propionate de clobétasol 500 µg/g seul (figures 1 et 2).

Les associations du propionate de clobétasol 500 µg/g avec le calcitriol (3 µg/g et 9 µg/g) n'ont pas montré une différence significative en terme d'efficacité comparée au propionate de clobétasol 500 µg/g seul (voir figure 2). L'ensemble des sept compositions testées a été bien toléré.

La composition comprenant 250 µg/g de propionate de clobétasol et 3 µg/g de calcitriol a montré des résultats meilleurs en terme de guérison (voir figures 3 et 4) par rapport :
- au propionate de clobétasol seul (250 µg/g),
- au propionate de clobétasol 250 µg/g en association avec 9 µg/g de calcitriol et,
- au Daivobet^{®} (Figure 3).

Les compositions comprenant respectivement une association :
- de 500 µg/g de propionate de clobétasol et de 3 µg/g de calcitriol et,
- de 500 µg/g de propionate de clobétasol et de 9 µg/g de calcitriol,
permettent d'obtenir des résultats sensiblement similaires à ceux obtenus avec 500 µg/g de propionate de clobétasol seul et supérieurs à ceux obtenus avec Daivobet^{®} (Figure 4).

## Revendications

1. Composition pharmaceutique destinée au traitement du psoriasis **caractérisée en ce qu'**elle comprend 250 µg de propionate de clobétasol par gramme de composition et 3 µg de calcitriol par gramme de composition pour une application une fois par jour sur la ou les parties de la peau affectée(s) par le psoriasis.

2. Composition selon la revendication 1, **caractérisée en ce qu'**elle comprend un ou plusieurs excipients pharmaceutiques adaptés pour une application topique.

3. Composition selon l'une quelconque des revendications 1 à 2, **caractérisée en ce qu'**elle se présente sous la forme d'un gel, d'une crème, d'une pommade, d'une lotion ou d'une solution.

4. Utilisation d'une association de propionate de clobétasol et de calcitriol pour la fabrication d'un médicament destiné au traitement du psoriasis, ledit médicament contenant une quantité de 250 µg de propionate de clobétasol et de 3 µg de calcitriol afin d'être appliqué une fois par jour sur la ou les parties de la peau affectée(s) par le psoriasis.

5. Utilisation selon la revendication 4, dans laquelle le médicament comprend un ou plusieurs excipients pharmaceutiques adaptés pour une application topique.

6. Utilisation selon l'une quelconque des revendications 4 et 5, dans laquelle le médicament se présente sous la forme d'un gel, d'une crème, d'une pommade, d'une lotion ou d'une solution.

## Claims

1. Pharmaceutical composition intended for the treatment of psoriasis, **characterized in that** it contains 250 µg of clobetasol propionate per gram of composition and 3 µg of calcitriol per gram of composition for once-per-day application to the part or parts of the skin affected by psoriasis.

2. Composition according to Claim 1, **characterized in that** it comprises one or more pharmaceutical excipients suitable for topical application.

3. Composition according to either of Claims 1 and 2, **characterized in that** it is in the form of a gel, cream, ointment, lotion or solution.

4. Use of a combination of clobetasol propionate and calcitriol for producing a medicinal product intended for the treatment of psoriasis, the said medicinal product containing an amount of 250 µg of clobetasol propionate and of 3 µg of calcitriol to be applied once per day to the part or parts of the skin affected by psoriasis.

5. Use according to Claim 4, wherein the medicinal product comprises one or more pharmaceutical excipients suitable for topical application.

6. Use according to either of Claims 4 and 5, wherein the medicinal product is in the form of a gel, cream, ointment, lotion or solution.

## Patentansprüche

1. Pharmazeutische Zusammensetzung, die für die Behandlung von Psoriasis vorgesehen ist, **dadurch gekennzeichnet, dass** sie für eine Applikation einmal täglich auf den oder die Bereiche der Haut, der (die) von Psoriasis betroffen ist (sind), 250 µg Clobetasolpropionat pro Gramm Zusammensetzung und 3 µg Calcitriol pro Gramm Zusammensetzung enthält.

2. Zusammensetzung nach Anspruch 1, **dadurch gekennzeichnet, dass** sie einen oder mehrere pharmazeutische Hilfsstoffe enthält, die für eine topische Anwendung geeignet sind.

3. Zusammensetzung nach einem der Ansprüche 1 bis 2, **dadurch gekennzeichnet, dass** sie in der Form eines Gels, einer Creme, einer Salbe, einer Lotion oder einer Lösung vorliegt.

4. Verwendung einer Kombination von Clobetasolpropionat und Calcitriol für die Herstellung eines Arzneimittels, das für die Behandlung von Psoriasis vorgesehen ist, wobei das Arzneimittel eine Menge von 250 µg Clobetasolpropionat und 3 µg Calcitriol enthält, um einmal täglich auf den oder die Bereiche der Haut, der (die) von Psoriasis betroffen ist (sind), aufgetragen zu werden.

5. Verwendung nach Anspruch 4, wobei das Arzneimittel einen oder mehrere pharmazeutische Hilfsstoffe enthält, die für eine topische Anwendung geeignet sind.

6. Verwendung nach einem der Ansprüche 4 und 5, **dadurch gekennzeichnet, dass** das Arzneimittel in der Form eines Gels, einer Creme, einer Salbe, einer Lotion oder einer Lösung vorliegt.
